# EUROPEAN PATENT APPLICATION

(11) **EP 1 579 886 A1**
(43) Date of publication of application: **28.09.2005**
(21) Application number: 03811511.9
(22) Date of filing: 11.11.2003
(51) Int. Cl.: A61M 29/02

(54) **MEDICAL INSTRUMENT FOR SOFT TISSUE AND METHOD FOR MANUFACTURE THEREOF**

(30) Priority: 21.11.2002 JP 2002337598
(71) Applicant: Independent Administrative Institute National Institute For Materials Science, Ibaraki 305-0047 (JP); Japan Lifeline Co., Ltd, Tokyo 171-0014 (JP)
(72) Inventor: KURODA, Daisuke, Tsukuba-shi, Ibaraki 305-0047 (JP); HANAWA, Takao, Tsukuba-shi, Ibaraki 305-0047 (JP); MAKINO, Morihide, Machida-shi, Tokyo 195-0053 (JP); KAWABATA, Takashi, Hasuda-shi, Saitama 349-0104 (JP)
(74) Representative: Wise, Stephen James
(86) International application number: PCT/JP2003/014307
(87) International publication number: WO 2004/045703

(57) **Abstract**

Ferritic stainless steel was produced by the melting method. The ferritic stainless steel was worked to the shape of a medical device for living soft tissue to obtain a medical device body. The medical device body was brought into contact with gas including nitrogen at a 800°C treatment temperature or more to make the ferritic stainless steel forming the medical device body absorb the nitrogen and transform at least part of the ferritic stainless steel to austenite.

## Description

### TECHNICAL FIELD

The present invention relates to circulatory system medical devices, digestive tract medical devices, and other medical devices for living soft tissue and methods for production of the same.

### BACKGROUND ART

Medical devices used for the cardiovascular system, including the cerebral circulatory system, etc. are required to have blood compatibility, but are easily improved by improvement of not only the materials, but also the surface properties etc. However, in long-term implanted medical devices, surface coatings easily break down leading to the appearance of the properties inherent to the base metal of the medical devices.

In the past, as the metal serving as the base of such medical devices, from the viewpoint of corrosion resistance, SUS304, SUS316, and other materials have been frequently used. For example, one type of medical device, a stent, is usually made of stainless steel (Japanese Patent Publication (A) No. 9 -117512, Japanese Patent Publication (A) No. 11-99207, Japanese National Disclosure (A) No. 12-501328, Japanese Patent Publication (A) No. 7-531, Japanese Patent Publication (A) No. 12-5321, etc.) Further, recently, there are known to have been attempts to make medical devices out of NiTi shape memory alloys.

However, there are reports that these metallic materials are problematic in the effect of the Ni contained on the body. At the present, metallic materials not including Ni and good in corrosion resistance in the body are being sought. However, in general, metallic materials superior in corrosion resistance are hard and poor in workability.

### DISCLOSURE OF THE INVENTION

The present invention was made in view of this situation and has as its object the economic provision of a medical device for living soft tissue not containing Ni, believed to be low in effect of the body, low in cytotoxicity, superior in workability, and superior in corrosion resistance, mechanical strength, and biocompatibility.

The inventors engaged in intensive study and as a result discovered that by producing ferritic stainless steel by the melting method, working it into a medical device for living soft tissue, then bringing it into contact with a gas containing nitrogen to make the nitrogen be absorbed into the worked product so as to transform at least part to austenite, it is possible to provide a delicate, complicated medical device for living soft tissue having superior workability, that this has superior corrosion resistance while maintaining sufficiently superior physical properties and practical properties, and that this has superior performance and safety as a medical device for living soft tissue, in particular a medical device for living soft tissue used in contact with the blood or in contact with blood vessels and thereby completed the present invention.

That is, the method for producing a medical device for living soft tissue of the invention has a melting step of producing ferritic stainless steel by the melting method, a working step of working said ferritic stainless steel to the shape of a medical device for living soft tissue to obtain a medical device body, and a nitrogen absorption step of bringing said medical device body into contact with a gas containing nitrogen at a predetermined treatment temperature or more to make said ferritic stainless steel forming said medical device body absorb nitrogen to transform at least part of said ferritic stainless steel to austenite.

The mechanical strength, modulus of elasticity, and other physical properties and the corrosion resistance are sharply improved by bringing the steel into contact with an inert gas containing nitrogen to make nitrogen be absorbed into the worked product so as to transform at least part to austenite. Further, the nitrogen is added to products made by ferritic stainless steel, so working is easier than with austenitic stainless steel and a product having the desired shape is easily obtained.

Therefore, a step requiring workability is performed by the material ferritic stainless steel, worked to a medical device for living soft tissue, then transformed to austenite to enable working to a complicated medical device for living soft tissue and provide a product superior in corrosion resistance and safety at a low cost.

Note that the above technique of bringing a medical device body worked to a desired shape into contact with an inert gas containing nitrogen gas at a predetermined temperature or more falls under the category of nitrogen absorption treatment classified as a so-called solid phase absorption method. By heating a medical device body to a predetermined temperature or more in a gas atmosphere containing nitrogen, nitrogen is added to all or part of the product.

Preferably, said ferritic stainless steel has as main ingredients Fe in an amount of 50 to 90 wt% (preferably 65 to 80 wt%), Cr and/or Mn in amounts of 10 to 30 wt% (preferably 15 to 25 wt%), and Mo and/or Ti in amounts of 0 to 10 wt% (preferably 0 to 5 wt%). The ferritic stainless steel preferably does not contain nickel.

Preferably, said treatment temperature is a temperature range of 800 to 1500°C, more preferably 1100 to 1300°C. If the treatment temperature is too low, the absorption of nitrogen in the ferritic stainless steel tends to become insufficient, while if the treatment temperature is too high, the physical properties tend to easily change.

Preferably, said ferritic stainless steel is made to contain nitrogen in an amount of at least 0.5 wt%, more preferably at least 0.8 wt%. If the amount of absorption of nitrogen in the ferritic stainless steel is too small, the effect of the present invention tends to become smaller.

Preferably, at least part of said ferritic stainless steel is made austenitic to form a two-phase structure of ferrite and austenite. Alternatively, all of said ferritic stainless steel is transformed to austenite. By forming a two-phase structure of ferrite and austenite or by transforming the entire amount to austenite, the corrosion resistance, mechanical strength, and biocompatibility are improved.

The medical device for living soft tissue according to the present invention is produced by the above method of production of the medical device for living soft tissue. The medical device for living soft tissue is not particularly limited, but the effect of the present invention is particularly great in the case of circulatory system medical devices or digestive tract medical devices. Circulatory system medical devices are medical devices used for example inside living organs of the circulatory system including the heart, central major blood vessels, peripheral blood vessels, and cerebral blood vessels or in contact with the outside walls of the organs. Further, digestive tract medical devices are medical devices used inside the stomach, intestines, and other parts of the digestive tract or in contact with the outside walls of the organs. This is because this type of medical device is often embedded in the body over a long period of time and the metal forming the medical devices preferably does not contain Ni. As specific examples of circulatory system medical devices, stents, stent grafts, vein filters, occlusion coils, materials for repairing abnormalities of the blood organs such as patent ductus arteriosus or atrial septal defects, artificial valves, blood vessel clips, etc. which are placed in the body for long periods of three months or more may be mentioned.

### BRIEF DESCRIPTION OF THE DRAWINGS

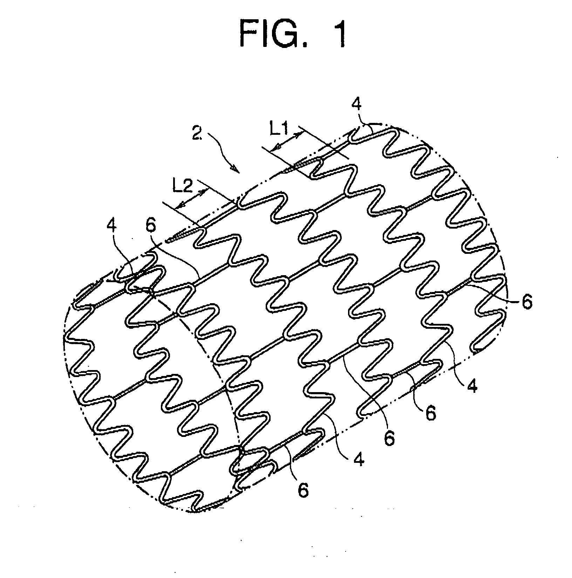

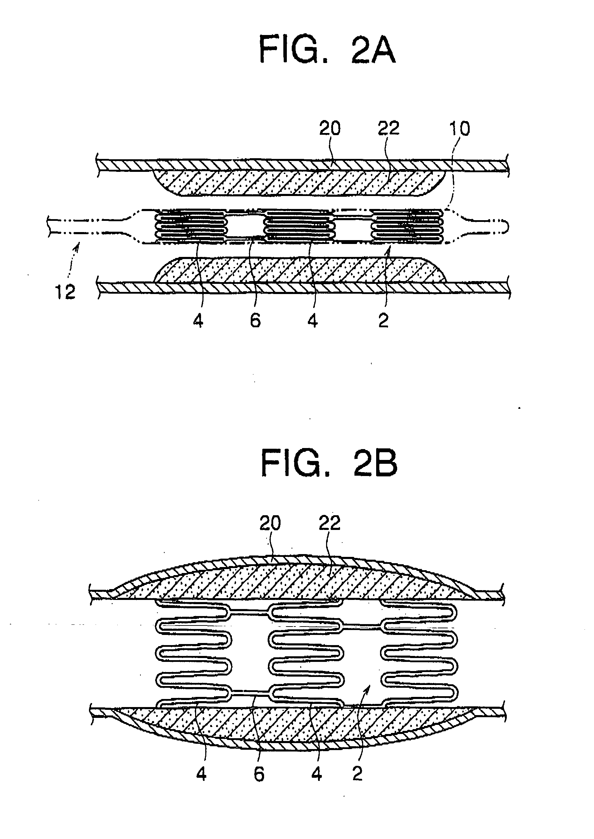

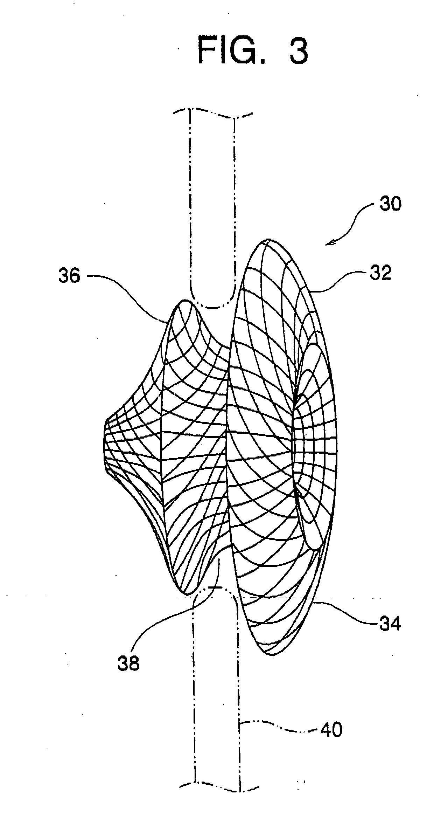

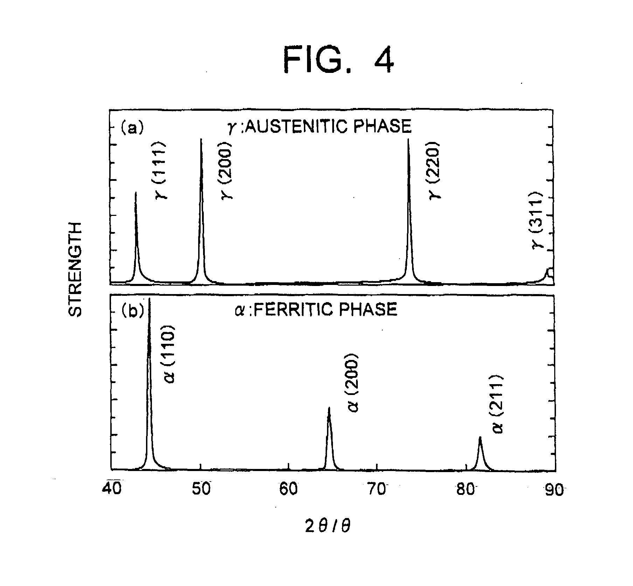

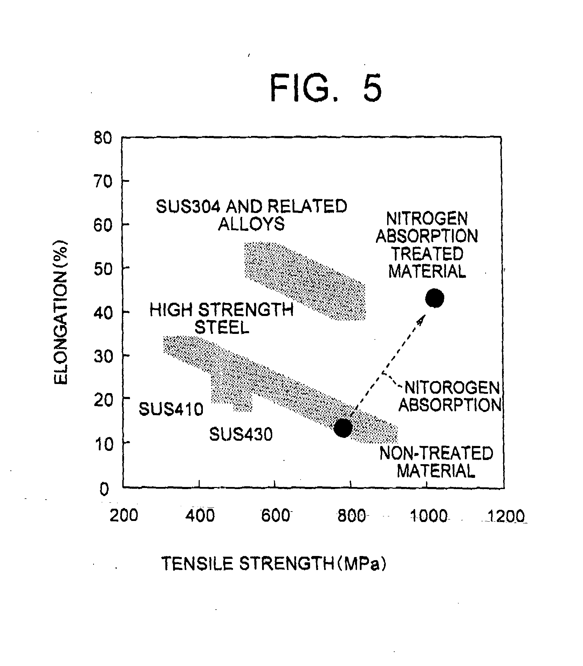

FIG. 1 is a schematic perspective view of a stent according to a first embodiment of the present invention. FIG. 2A and FIG. 2B are cross-sectional views of principal parts showing the state of use of a stent. FIG. 3 is a schematic perspective view of a prosthesis according to another embodiment of the present invention. FIG. 4A is an X-ray diffraction pattern of a test piece after nitrogen absorption treatment, while FIG. 4B is an X-ray diffraction pattern of a test piece equivalent not subjected to nitrogen absorption treatment. FIG. 5 is a graph of the correlation showing the balance between strength and ductility between a test piece after nitrogen absorption treatment and a test piece equivalent not subjected to nitrogen absorption treatment.

### BEST MODE FOR WORKING THE INVENTION

Below, the present invention will be explained in detail based on the embodiments shown in the figures.

### First Embodiment

As shown in FIG. 1, the stent 2 serving as a circulatory system medical device according to the present embodiment is a substantially cylindrically shaped stent placed in its entirety in an arterial cavity of the body and has first stent elements 4 and second stent elements 6. The first stent elements 4 and the second stent elements 6 form the medical device body.

The first stent elements 4 are present along the circumferential direction and have shapes expandable in outside diameter. They are made of materials resistant to collapse after expansion of the outside diameter. The shape enabling expansion of the outside diameter is not particularly limited, but specifically may be, along the circumferential direction, a wave shape, peak-valley shape, a sine/cosine curve shape, a zigzag shape, a string-of-pearl shape, a sawtooth shape, a pulse shape, or a combination of the same or other repeating shapes etc. The material resistant to collapse after expansion of its outside diameter, in the present embodiment, is made ferritic stainless steel brought into contact with a gas containing nitrogen at a predetermined treatment temperature or more to make it absorb nitrogen to transform at least part to austenite.

The second stent elements 6 are stent elements for connecting in the axial direction a plurality of said first stent elements 4 arranged in the axial direction. In the present embodiment, they are comprised of the same material as the first stent elements.

The width and/or thickness of the first stent elements 4 is preferably 30 to 400 µm, more preferably 50 to 100 µm, while the width and/or thickness of the second stent elements 6 is preferably 20 to 100 µm, more preferably 30 to 60 µm. The axial direction length L1 of the repeating units forming the first stent elements 4 is not particularly limited, but preferably is 0.5 to 5 mm, more preferably 0.8 to 2 mm. The axial direction length L2 of the second stent elements 6 is not particularly limited, but preferably is 0.5 to 5 mm, more preferably 1.5 to 3 mm. Note that the second stent elements 6 do not necessarily have to be straight lines parallel to the center axis of the stent and may also be slanted lines, curves, or combinations of the same.

In the present embodiment, the second stent elements 6 are arranged more sparsely along the circumferential direction than the first stent elements 4. For example, it is preferable to arranging two to six second stent elements 6 in the circumferential direction of the first stent elements 4. Further, the second stent elements 6 may connect the peak parts of the repeating units of the first stent elements 4 or may connect the valley parts or the peak parts and the valley parts. Further, they may also connect the parts in the middles of the peaks and valleys.

The dimensions of the stent 2 as a whole are suitably determined in accordance with the objective of use etc. and are not particularly limited, but for example when used for treatment of a coronary artery, the outside diameter of the stent 2 at the time of expansion is preferably 2 to 5 mm and the axial direction length is 15 to 40 mm. Further, in the case of a stent for treatment of a peripheral blood vessel, the outside diameter at the time of expansion of the stent 2 is preferably 3 to 10 mm, and the axial direction length is 15 to 40 mm. Further, in the case of a stent for treatment of the aorta, the outside diameter at the time of expansion of the stent 2 is preferably 5 to 30 mm, and the axial direction length is 30 to 100 mm.

The surfaces of the first stent elements 4 and second stent elements 6 forming the stent 2 may be covered by a plating film and/or biocompatible coating film. This is for improving the biocompatibility. Further, as a plating film, a platinum or gold plating film is used. The biocompatible coating film is not particularly limited, but for example a polyethylene or other olefin, polyimide or polyamide or other nitrogen-containing polymer, siloxane polymer, or other ordinary polymer used for medical purposes etc. may be used. Further, the coating film is not limited to a polymer and may also be an inorganic coating film of silicon carbide, pyrolitic carbon, diamond-like carbon, or other carbon etc. Further, the surface of the stent 2 may also be treated to be made hydrophilic. The surface of the stent 2 may also have immobilized at it an enzyme, bio ingredient, or chemical for preventing reconstriction. The film thicknesses are not particularly limited, but the thickness of the plating film is for example 0.05 to 5 µm or so, while the thickness of the biocompatible coating film is 0.1 to 10 µm or so, preferably 0.5 to 5 µm.

Next, the method of production of the stent of the present embodiment will be explained.

First, the ferritic stainless steel serving as the material of the medical device body comprised of the first stent elements 4 and second stent elements 6 is produced. This ferritic stainless steel is produced by the melting method. The "melting method" means the process of placing the materials forming the ingot into a furnace and melting them to make them homogeneous. The conditions at the time of melting are not particularly limited, but preferably the furnace is heated in the vacuum state up to near 1600°C and then the materials are added.

Next, the ferritic stainless steel produced by melting method is worked to form a metal tube (thickness of 50 to 400 µm).

Next, the metal tube is coated on its surface with a photosensitive cross-linkable resist and is exposed by a reduction projection exposure apparatus using a previously prepared master as a photomask to transfer the master pattern to the resist. At this time, the exposure is performed while rotating the metal tube. After this, an ordinary method is used to dissolve away the uncross-linked parts of the resist to form patterns of first stent elements 4 and second stent elements 6 on the resist, then the unnecessary metal parts are removed by etching to obtain the medical device body. This medical device body is comprised of ferritic stainless steel produced by melting method.

Next, this medical device body is brought into contact with an inert gas containing nitrogen at a predetermined treatment temperature or more to make the ferritic stainless steel forming the medical device body absorb nitrogen and transform at least part of, preferably all of, the ferritic stainless steel to austenite, whereby the stent 2 shown in FIG. 1 is obtained.

Next, an example of use of the stent of the present embodiment will be explained. As shown in FIG. 2A, the stent 2 is first fit over the circumference of a balloon part 10 of a balloon catheter 12 in the state compressed in the radial direction. In that state, the balloon catheter 12 is inserted into a blood vessel 20 or other body cavity. After this, the stent is passed together with the balloon part 10 of the balloon catheter 12 through the inside of the blood vessel 20 bending by up to 90 degrees or more and finally reaches a constricted part 22 of the blood vessel 20. In the stent 2 according to the present embodiment, mainly the second stent elements 6 easily bend to match the bent shape of the blood vessel 20 and return to their original shapes after positioning at the target constricted part 22. Therefore, the ability of the stent 2 to follow bends and the insertion properties are improved.

After this, as shown in FIG. 2B, the constricted part 22 expands together with the expansion of the balloon part 10. The stent 2 also simultaneously expands outward in the radial direction. After this, only the balloon catheter 12 is pulled out from the blood vessel 20 so as to leave only the expanded stent 2 inside the expanded constricted part 22 and prevent reconstriction. In the present embodiment, the first stent elements 4 in the stent 2 are parts for countering the force trying to return the constricted part after expansion to its original form. They are made of materials not easily collapsing, so can effectively prevent reconstriction.

Further, in the present embodiment, the material is worked to a medical device body, then the worked product is made to absorb nitrogen so as to transform at least part to austenite. Therefore, by performing a step requiring workability by the ferritic stainless steel material, working this to a medical device, then transforming this to austenite, it becomes possible to provide a product able to be worked to a complicated medical device and superior in corrosion resistance and safety.

Note that the specific form of the stent 2 is not limited to the one shown in FIG. 1. A spiral shape, a hexagonal single-layer stack structure, a hexagonal double-layer stack structure, and various other shapes may also be considered.

### Second Embodiment

As shown in FIG. 3, a prosthesis 30 serving as a circulatory system medical device according to the present embodiment has wires 32 forming a mesh. By braiding the wires 32, as shown in FIG. 3, a first disk 34 and second disk 36 and a recess 38 formed between the two are formed whereby overall an hourglass shaped medical device body is formed. FIG. 3 shows the state where the prothesis 30 is expanded. It is designed to fur example plug a hole naturally formed in a body wall 40. The prosthesis 30 in the state before passing through the hole of the body wall 40 is transported to the hole of the body wall 40 by a catheter etc. in the state compressed in outside diameter and then expanded there.

In the present embodiment, the wires 32 forming the medical device body, in the same way as the first embodiment, are comprised of ferritic stainless steel brought into contact with a gas containing nitrogen at a predetermined treatment temperature or more to make it absorb nitrogen and transform at least part to austenite.

In the present embodiment, the material is worked to the medical device body, then the worked product is made to absorb nitrogen so as to transform at least part to austenite. Therefore, by performing a step requiring workability by the ferritic stainless steel material, working this to a medical device, then transforming this to austenite, it becomes possible to provide a product able to be worked to a complicated medical device and superior in corrosion resistance and safety.

Note that the present invention is not limited to the above-mentioned embodiments and can be modified in various ways within the range of the present invention.

For example, the medical device is not limited to the illustrated embodiments. Various medical devices for living soft tissue may be considered.

Below, the present invention will be explained further based on detailed examples, but the present invention is not limited to these examples.

### Example 1 and Comparative Example 1

A vacuum arc melting furnace was used to first produce a 3.5 kg ingot of ferritic stainless steel (24 wt% of Cr, 2 wt% of Mo, and the balance of substantially Fe). This was produced by heating the furnace in a vacuum state to 1600°C and then adding the materials.

This ingot was divided into four and cut to obtain 25 mm x 25 mm x 110 mm blocks, which were then hot forged at 1100°C and cold forged at room temperature to prepare rods of a diameter of 9 mm x 90 mm and sheets of a thickness of 1.5 mm x 15 mm x 15 mm. These rods were further machined to prepare rod tensile test pieces. These two types of test pieces were subjected nitrogen absorption treatment as explained below using a nitridation system.

That is, each test piece was placed on a SUS304 mesh board, degreased and washed by acetone, then inserted and arranged into a nitridation part of a nitridation system which was then evacuated to a vacuum to 2 Pa by a rotary pump. Next, the nitridation part was charged at a flow rate of 2 L/min with an inert gas containing nitrogen gas. The nitridation part was raised from room temperature at a rate of 5°C/min to 1200°C and the test piece and nitrogen gas were brought into contact at 1200°C for 24 hours.

After the above nitrogen absorption treatment, the test piece was quenched from 1200°C in ice water. The surface scale was removed by polishing, then an X-ray diffraction system was used to identify the microstructure. In this X-ray diffraction, a CuKα tube was used to change from 2θ/θ=40° to 90° by 1°/min increments. FIG. 4A shows the obtained X-ray diffraction pattern. For comparison, FIG. 4B shows the X-ray diffraction pattern of a test piece equivalent not subjected to nitrogen absorption treatment. As will be clear from a comparison with FIGS. 4A and 4B, it was confirmed that the test piece after nitrogen absorption treatment became completely austenitic stainless steel. The amount of addition of nitrogen was about 0.9 wt%.

Next, a 100kN capacity Instron-type tensile tester was used to conduct a tensile test on the test piece at a crosshead speed of 0.5 mm/min. The balance of the strength and ductility of a test piece after nitrogen absorption treatment, a test piece equivalent not subjected to nitrogen absorption treatment, and existing alloy is shown in FIG. 5. As confirmed from FIG. 5, if performing nitrogen absorption treatment, compared with existing alloy and a test piece not subjected to nitrogen absorption treatment, the balance of strength and ductility was superior. This tendency was confirmed in the same way as the case where the test piece was a cold rolled material not subjected to nitrogen absorption treatment. The efficacy of nitrogen absorption treatment was reconfirmed.

Further, each test piece was also evaluated for corrosion resistance. Test pieces of the example of the invention, SUS316L stainless steel test pieces, and test piece equivalents not subjected to nitrogen absorption treatment piece were dipped in different test solutions adjusted to 37°C and deaerated using nitrogen, that is, a 0.9% NaCl solution, PBS(-) solution, Hanks solution, and Eagle's MEM solution.

For each test solution, the test piece subjected to the nitrogen absorption treatment could be confirmed to exhibit a superior corrosion resistance compared with SUS316L stainless steel and a test piece not subjected to nitrogen absorption treatment. That is, the test pieces of the example of the invention were not observed to have any pitting, while the SUS316L stainless steel suffered from pitting.

### Example 2

In the same way as Example 1, steel sheet of a thickness of 1.5 mm x 15 mm x 15 mm was prepared, then the steel sheet was rolled up and welded to form a tube shape. This was further worked to form a tube of a thickness of 100 µm and an outside diameter of 1.5 mm.

This tube was cut by a YAG laser, fetomsecond laser, etc. and chemically polished to obtain a stent 2 of the shape shown in FIG. 1. This stent 2 was degreased and washed by acetone, then brought into contact with nitrogen gas at 1200°C for 10 minutes. The nitrogen gas contact conditions at this time were the same as Example 1.

This stent was mounted over a 3F polyamide balloon catheter (1 mm outside diameter of balloon at the time of expansion) and folded to give a stent outside diameter of 1.2 mm to reduce its size.

Using the balloon, the folded stent was expanded by the balloon catheter by a contrast agent solution and the expansion pressure at that time measured. In this example, the expansion pressure was 8 Pa. It could be confirmed that expansion was possible with a relatively low expansion pressure.

Further, the stent of this example was expanded to an outside diameter of 3 mm, then subjected to a water pressure of 10 atm using a cylindrical balloon to examine collapsing, but no abnormalities were observed. The stent withstood even pressurization to 20 atm and had durability sufficient for practical use. Further, it was placed in a pressurizing tube and measured for pressure and recoil. Even at 2 atm, there was only slight recoil and a sufficient strength was exhibited. Further, it was confirmed that after expansion of the stent, it was not easily crushed.

Further, the stent was mounted at a stent delivery catheter and inserted into a 3φ simulated circuit having three bends of R =10, 10, 20. Smooth insertion was possible and there was no problem. Further, it was confirmed that at the straight parts after the bent parts, the stent was expanded, but no large looseness was seen.

### Comparative Example 2

Other than not performing the nitrogen absorption treatment, the same procedure was performed as in Example 2 to prepare a stent which was then tested in the same way as Example 2. The expansion pressure was 8 Pa or similar to the examples, but at a water pressure of 10 atm, the stent ended up collapsing or crushing was also observed and there were problems in terms of mechanical strength.

As explained above, according to the present invention, it is possible to economically provide a medical device for living soft tissue not containing Ni, believed to be low in effect of the body, low in cytotoxicity, superior in workability, and superior in corrosion resistance, mechanical strength, and biocompatibility.

## Claims

1. A method for producing a medical device for living soft tissue having:
a melting step of producing ferritic stainless steel by melting method,
a working step of working said ferritic stainless steel to the shape of a medical device for living soft tissue to obtain a medical device body, and
a nitrogen absorption step of bringing said medical device body into contact with a gas containing nitrogen at a predetermined treatment temperature or more to make said ferritic stainless steel forming said medical device body absorb nitrogen to transform at least part of said ferritic stainless steel to austenite.

2. The method of production of a medical device for living soft tissue as set forth in claim 1, wherein said ferritic stainless steel has as main ingredients Fe in an amount of 50 to 90 wt%, Cr and/or Mn in amounts of 10 to 30 wt%, and Mo and/or Ti in amounts of 0 to 10 wt%.

3. The method of production of a medical device for living soft tissue as set forth in claim 1, wherein said ferritic stainless steel has as main ingredients Fe in an amount of 65 to 80 wt%, Cr and/or Mn in amounts of 15 to 25 wt%, and Mo and/or Ti in amounts of 0 to 5 wt%.

4. The method of production of a medical device for living soft tissue as set forth in any one of claims 1 to 3, wherein said treatment temperature is in a temperature range of 800 to 1500°C.

5. The method of production of a medical device for living soft tissue as set forth in any one of claims 1 to 3, wherein said treatment temperature is in the temperature range of 1100 to 1300°C.

6. The method of production of a medical device for living soft tissue as set forth in any one of claims 1 to 5, wherein said ferritic stainless steel is made to contain nitrogen in an amount of at least 0.5 wt%.

7. The method of production of a medical device for living soft tissue as set forth in any one of claims 1 to 5, wherein said ferritic stainless steel is made to contain nitrogen in an amount of at least 0.8 wt%.

8. The method of production of a medical device for living soft tissue as set forth in any one of claims 1 to 7, wherein at least part of said ferritic stainless steel is transformed to austenite to form a two-phase structure of ferrite and austenite.

9. The method of production of a medical device for living soft tissue as set forth in any one of claims 1 to 7, wherein all of said ferritic stainless steel is transformed to austenite.

10. The medical device for living soft tissue produced by a method of production of a medical device for living soft tissue as set forth in any one of claims 1 to 9.
